# EUROPÄISCHE PATENTSCHRIFT

(11) **EP 1 075 650 B1**
(45) Veröffentlichungstag und Bekanntmachung des Hinweises auf die Patenterteilung: **07.06.2006**
(21) Anmeldenummer: 99920802.8
(22) Anmeldetag: 27.04.1999
(51) Int. Cl.: G01N 19/04, G01N 33/32, B41F 7/00, B41F 13/02, B41F 9/04

(54) **VORRICHTUNG UND VERFAHREN ZUR MESSUNG DER KLEBRIGKEIT EINES FLIESSFÄHIGEN MEDIUMS**
DEVICE AND METHOD FOR MEASURING THE STICKINESS OF A FLOWABLE MEDIUM
DISPOSITIF ET METHODE POUR MESURER L'ADHESIVITE D'UNE SUBSTANCE COULANTE

(30) Priorität: 30.04.1998 DE 19819455
(43) Veröffentlichungstag der Anmeldung: 14.02.2001
(73) Patentinhaber: prüfbau Dr.-Ing. H. Dürner GmbH, 82380 Peissenberg (DE)
(72) Erfinder: BAUERNSCHMID, Peter, D-83317 Teisendorf (DE)
(74) Vertreter: Zmyj, Erwin
(86) Internationale Anmeldenummer: PCT/EP1999/002850
(87) Internationale Veröffentlichungsnummer: WO 1999/057540

(56) Entgegenhaltungen:
- EP-A- 0 109 725
- EP-A- 0 741 295
- NL-A- 8 600 912
- US-A- 3 901 149

## Beschreibung

Die Erfindung bezieht sich auf eine Vorrichtung und ein Verfahren zur Messung der Klebrigkeit eines fließfähigen Mediums, insbesondere einer Druckfarbe, das als Schicht zwischen zwei in gleicher Richtung bewegbaren Flächen durch eine Konvergenzzone, eine Druckzone und eine Divergenzzone geführt wird, wobei als Maß für die Klebrigkeit diejenige Kraft mittels einer Messvorrichtung messbar ist, die notwendig ist, um die Flächen voneinander zu trennen.

Der Begriff Klebrigkeit soll dabei insbesondere die Zügigkeit und sonstige rheologische Eigenschaften, wie z.B. Viskosität umfassen.

Bei einer aus der DE-A-19 00 328 bekannten Vorrichtung dieser Art wird das zu untersuchende, fließfähige Medium in genau dosierter Menge zwischen zwei drehbare Körper gebracht, von denen der eine angetrieben und der andere durch Reibschluss mitgeführt wird. Dabei wird über eine Hebelanordnung die Kraft gemessen, die notwendig ist, um die beiden Körper voneinander zu trennen. Diese Vorrichtung weist den grundsätzlichen Nachteil auf, dass durch den Antrieb nur eines drehbaren Körpers und die Mitnahme des zweiten drehbaren Körpers über das zu messende fließfähige Medium, in diesem Medium Scherkräfte auftreten, die zu einer Veränderung der rheologischen Eigenschaften des zu untersuchenden Mediums führen, wodurch die für die Bestimmung der Zügigkeit definierte Kraft, die bei ihrer Messung senkrecht zu den beiden Flächen stehen soll, zwischen denen sich das zu messende Medium befindet, verfälscht wird. Ein weiterer Nachteil dieser und anderer bekannter Vorrichtungen besteht grundsätzlich darin, dass bei der Prüfung von Druckfarben und deren lithographischen Eigenschaften diejenigen Einflüsse nicht berücksichtigt werden können, die sich beispielsweise beim Auftragen des fließfähigen Mediums, z. B. einer Druckfarbe auf Papier oder sonstigen Materialien ergeben, weil die Vorrichtung aus zwei rotierenden Körpern mit Metall- und/oder Gummioberflächen bestehen, dass aber beispielsweise die in einer Druckmaschine herrschenden Voraussetzungen mit einer solchen Vorrichtung nicht nachgestellt werden können. Weiterhin ist es auch nicht möglich den Einfluss der Schichtdicke des aufgetragenen Mediums in Abhängigkeit vom Material der Flächen auf den Zügigkeitswert zu ermitteln. Ebenso besteht keine Möglichkeit die bei einem Mehrfarbendruck erfolgende Überlagerung verschiedener fließfähiger Medien hinsichtlich ihres Einflusses auf die Zügigkeit festzustellen. Alle solche Untersuchungsmöglichkeiten sind mit dieser bekannten Vorrichtung und auch weiteren bekannt gewordenen Vorrichtungen nicht möglich.

Eine weitere bekannte Vorrichtung, die nach dem gleichen Grundprinzip arbeitet, wie die bereits erläuterte Vorrichtung, ist aus der DE-A-37 11 864 bekannt. Bei dieser bekannte Vorrichtung wird das hinsichtlich der Klebrigkeit zu messende fließfähige Medium zwischen eine angetriebene Walze und eine gegen die Kraft einer Feder verschiebbare Walze gebracht und diejenige Kraft gemessen, die notwendig ist, um die beiden Walzen voneinander zu trennen, wobei für die Messung Dehnungsmessstreifen in der Feder angeordnet sind.

Bei einer weiterhin aus der US-A-3901149 bekannten Vorrichtung wird das zu messende Medium zwischen zwei divergierenden Riemenflächen gebracht und eine der Riemen angetrieben, wodurch über das Medium der zweite Riemen mitgenommen wird. Das zu messende Medium versucht die beiden Riemen zusammenzuhalten, wobei elastische Mittel vorgesehen sind, um die beiden Riemen auf eine vorbestimmte Spannung zu halten, wodurch die durch die klebrigkeitsverursachte Länge des Zusammenhaftens beider Riemen als Maß der Klebrigkeit gemessen werden kann.

Aufgabe der Erfindung ist es, eine Vorrichtung und ein Verfahren der eingangs erläuterten Art so auszugestalten, dass die beim Auftragen, Übertragen, mehrmaligen Überdecken, bei der Veränderung durch Zugabe weiterer Stoffe und bei der Aufbereitung von fließfähigen Medien, in verschiedenen Bereichen, beispielsweise in Verreibewerken, am Druckzylinder oder am Bedruckstoff von Druckereimaschinen auftretenden Klebrigkeitswerte und insbesondere Zügigkeitswerte und deren Veränderungen entsprechend den tatsächlich auftretenden Verhältnissen gemessen werden können.

Diese Aufgabe wird mit einer Vorrichtung gemäß Anspruch 5, bzw. mit einem Verfahren gemäß Anspruch 1, gelöst.

Eine wesentliche Ausgestaltung der Vorrichtung nach der Erfindung besteht darin, dass zwei Flächen in gleicher Richtung mit gleicher Geschwindigkeit antreibbar sind, so dass das zwischen diesen Flächen befindliche, zu untersuchende, fließfähige Medium keinen nennenswerten Scherkräften unterworfen wird, die zu einer Änderung der rheologischen Eigenschaften dieses fließfähigen Mediums führen könnten. Um diese gleichen Geschwindigkeiten zu erreichen, ist es erforderlich, dass eine der Flächen auf einer kreiszylindrischen Bahn geführt ist, d.h. diese Fläche Teil eines Kreiszylinders ist. Die zweite Fläche kann zwar eben sein, muss aber auch mit der gleichen Geschwindigkeit bewegt werden können, die der Umfangsgeschwindigkeit des Kreiszylinders entspricht. Selbstverständlich ist es für die kontinuierliche Messung der Zügigkeitswerte vorteilhaft, wenn beide Flächen auf kreiszylindrischen Bahnen geführt sind, da hierdurch die Zeitunterbrechung für die berührungslose Rückführung der ebenen Fläche in die Ausgangslage entfällt.

Ein weiteres entscheidendes Merkmal der vorliegenden Erfindung ist in der Tatsache zu sehen, daß die mit dem fließfähigen Medium während des Meßvorganges in Berührung stehenden Flächen denjeningen Flächen hinsichtlich der Materialauswahl entsprechen, die bei den Vorrichtungen zum Einsatz kommen, in denen das zu messende, fließfähige Medium im bestimmungsgemäßen Gebrauch verwendet wird. Beispielsweise besteht eine Fläche aus dem zu bedruckenden Papier oder einem sonstigen zu bedruckenden Werkstoff und die andere Fläche besteht aus dem Material, aus dem auch die in einer Druckmaschine eingesetzte Druckwalze oder sonstige Druckvorrichtung besteht. Hierdurch werden exakt diejenigen Verhältnisse nachgestellt, wie sie auch in der anwendungsgemäßen Vorrichtung, d.h. in einer Druckereimaschine vorliegen. Für den Fachmann ist es notwendig zu wissen, wie sich eine Druckfarbe während des Druckens verhält, wo bestimmte Inhaltsstoffe dieser Druckfarbe, z.B. Öle, Wasser, von dem zu bedruckenden Material, d.h. beispielsweise dem Papier, aufgenommen werden wodurch diese Druckfarbe in ihrer rheologischen Eigenschaft verändert wird. Bei den bisher üblichen Meßvorrichtung, bei denen die aus den bestimmungsgemäßen Vorrichtungen vorliegenden Materialien nicht zum Einsatz kommen, können auch diese entsprechenden Eigenschaften und Veränderungen des fließfähigen Mediums nicht festgestellt werden. So kann beispielsweise bei einer Meßvorrichtung, die aus einer Stahlwalze und einer Gummiwalze besteht, niemals der Effekt auftreten, wie er bei der Verwendung einer Papieroberfläche eintritt, an der das zu untersuchende, fließfähige Medium während des Meßvorganges haftet.

Schließlich ist es auch besonders bedeutungsvoll, daß die Meßeinrichtungen, welche zur Feststellung der Größe und der Richtung der auftretenden Kräfte verwendet werden, mit den Flächen mitbewegt werden, an denen die Kräfte auftreten. Hierdurch wird über den Meßübermittler auf die jeweiligen zugeordneten Meßeinrichtungen, die sich aus dem Anhaften des zu untersuchenden Mediums an der Fläche und den beim Trennvorgang des Mediums von dieser Fläche ergebenden Kräfte an jeder Stelle des anhaftenden Bereichs erfaßt, so daß der vollständige Ablauf eines Ablöse- oder Spaltvorganges hinsichtlich Kraftgröße und Kraftrichtung erfaßt werden kann. Da die Meßeinrichtungen mitbewegt werden, können sie kontinuierlich die aufgenommenen Werte auf Auswerteeinrichtungen übertragen, ohne daß Veränderungen in dem zu untersuchenden Medium durch irgendwelche Ruhepausen eintreten, die bei manchen bekannten Meßvorrichtungen zwangsläufig unvermeidbar sind. Die Meßvorrichtungen sind also durch diese erfindungsgemäße Ausgestaltung sozusagen stets am Ort des Geschehens und können somit den gesamten Veränderungsbereich der rheologischen Eigenschaften während des Spaltens aufnehmen. Selbstverständlich ist es bei diesem Meßvorgang erforderlich, daß die betrachtete Fläche, an welcher das zu untersuchende Medium anhaftet, mindestens einem dehnfähigen Bereich in Verbindung steht, wobei es gleichgültig ist, ob diese Fläche selbst dehnfähig ist oder die Dehnfähigkeit im Meßübermittler an irgendeiner Stelle desselben, also entweder im Endbereich oder in den dazwischen liegenden Bereichen ausgebildet ist.

Wenn eingangs von der Messung der Klebrigkeit eines fließfähigen Mediums gesprochen wird, so sollen hierdurch sämtliche Arten rheologischer Eigenschaften verstanden werden. Bei der Erfassung der Zügigkeit eines fließfähigen Mediums wird man nur die senkrecht zur Fläche beim Ablöse- bzw. Spaltvorgang des Mediums auftretende Kraft messen. Stellt man aber die unter einem Winkel verlaufenden Kräfte fest, die sich gegen Ende des Spaltvorganges einstellen, so kann man hier andere Eigenschaften, beispielsweise die Viskosität des Mediums, beurteilen. Mit dem Begriff "Klebrigkeit soll also ein gewisser Oberbegriff zum Ausdruck gebracht werden, der selbstverständlich die Zügigkeit als einen bevorzugten Sonderfall umfaßt.

Um die Vorrichtung auf die verschiedenen zu überprüfenden Materialien einstellen zu können, ist es vorteilhaft, wenn in weiterer Ausgestaltung der Erfindung die Flächen in ihrem gegenseitigen Abstand und hinsichtlich der Bewegungsgeschwindigkeit einstellbar sind. Hierdurch lassen sich auch kontrollierte Scherkräfte erzeugen, um praxisnahe Verhältnisse zu schaffen. Obwohl die Anordnung der Meßeinrichtung unabhängig von der Form der Stützkörper ist, so ist es doch für die kontinuierliche Messung vorteilhaft, wenn die als Meßübermittler dienende Fläche auf einem zylindrischen Stützkörper aufgebracht ist. Ein zylindrischer Stützkörper mit einer ebenso zylindrischen Fläche ist für die Aufnahme aufeinanderfolgenden Meßdaten und insbesondere für die kontinuierliche Überprüfung des Mediums wesentlich besser geeignet als eine linear bewegte Fläche, die immer wieder an ihren Ausgangsort zurückgebracht werden muß.

Wenn in weiterer Ausgestaltung der Erfindung mehrere bewegbare Flächen der die Kräfte aufnehmenden und auf Meßeinrichtungen übermittelnden Fläche zugeordnet sind, so können hierdurch entweder die Vorgänge bei einem Mehrfarbendruck simuliert werden, bei dem mehrere fließfähige Medien unterschiedlicher rheologischer Eigenschaften übereinandergeschichtet werden oder es können bei ein und demselben Medium die Vorgänge des Rückspaltens überprüft werden, d.h. diejenigen Vorgänge überprüft werden, die beim mehrmaligen aufeinanderfolgenden Spalten des zu untersuchenden Materials in einem divergierenden Bereich zwischen zwei bewegten Flächen auftreten. Mit dieser Anordnung ist es auch möglich, die Auswirkungen des Auftragens von Zusatzstoffen, beispielsweise von Wasser auf das fließfähige Medium zu untersuchen. Dieser Auftrag ist mit einer dieser mehreren zusätzlichen bewegten Flächen möglich.

Hierbei ist es besonders vorteilhaft, wenn die weiteren zugeordneten Flächen kreiszylindrisch ausgebildet sind, da die Einwirkungen auf die zu messende Schicht des fließfähigen Mediums viel besser mittels rotierender Flächen ausgeübt werden können als mit ebenen bewegten Flächen.

Eine grundsätzliche Ausgestaltung der Anordnung zur Erfassung der Kräfte besteht nach der Erfindung darin, daß die Meßeinrichtungen zur Messung der Kraft zwischen den Flächen hinsichtlich Größe und Richtung mechanisch mit der Fläche oder dem Meßübermittler in Verbindung stehen. Hier wird also zwischen der Fläche oder dem Meßübermittler und der Meßeinrichtung eine mechanische Verbindung eingesetzt, die diese an der Fläche angreifenden Kräfte auf die Meßeinrichtung überträgt.

Wenn mehrere mechanische Verbindungen mit der Fläche oder dem Meßübermittler mit Abstand zueinander in Bewegungsrichtung gesehen, ausgebildet sind, dann lassen sich aufeinanderfolgende Schritte in der Entwicklung des Kraftaufbaus während des Spaltvorganges darstellen.

Es ist auch möglich, eine optische Meßeinrichtung innerhalb eines Stützkörpers zur Feststellung der räumlichen Auslenkung des Meßübermittlers vorzusehen. Hier können optische Meßeinrichtungen beispielsweise durch entsprechende Bohrungen in der Wand des Stützkörpers die sich ändernden Abstände des Meßübermittlers gegenüber dem Stützkörper feststellen.

Wenn in weiterer Ausgestaltung der Erfindung zwei kreiszylindrische Stützkörper mit auslenkbaren Flächen und einem Meßwertübermittler, die zusammen mit den Meßeinrichtungen ein Meßwerk bilden, vorgesehen sind, die zwischen sich das zu untersuchende Medium aufnehmen, so können verhältnismäßig lange nahezu parallele Strecken zwischen zwei einander gegenüberliegenden Meßwertübermittlern gebildet werden, in denen sich das zu überprüfende fließfähige Medium befindet. Hierdurch ist es insbesondere im Zusammenhang mit einer weiteren Ausgestaltung der Erfindung, bei der die auslenkbare Fläche aus einem das Medium unbeeinflußenden Material bestehen, möglich, die durch äußere Einflüsse unbeeinflußte dem zu messenden Medium innewohnende rheologische Eigenschaft, insbesondere die Zügigkeit zu messen, da der Spaltvorgang an beiden Flächen, an denen das Medium anhaftet über eine verhältnismäßig lange Strecke hinsichtlich der Kraftentfaltung gemessen werden kann. Hierdurch ist die dem Material innewohnende und durch keine äußeren Einflüsse beeinflußte sogenannten "Urzügigkeit" feststellbar.

Wenn man die rheologischen Eigenschaften eines fließfähigen Mediums nicht nur innerhalb eines Spaltes zwischen zwei bewegten Flächen, sondern auch bei Einwirkung von Scherkräften feststellen will, wie das beim Changieren von Walzen in einem Farbwerk oder Verreibewerk auftritt, so ist es empfehlenswert die Vorrichtung so auszugestalten, daß zwischen den beiden aus Stützkörper und Meßwertübermittlern ausgebildeten Meßwerken eine Walze angeordnet ist, die mit beiden Meßwerken zusammenwirkt und zusätzlichen in ihrer Achsrichtung hin- und herverschiebbar antreibbar ist. Hierbei werden den im divergierenden Bereich auftretenden Spaltkräfte auch noch durch die Changierbewegung hevorgerufende Scherkräfte überlagert, die in das Meßergebnis eingehen und eine Wirklichkeitsnahe Erfassung der Kräfte hinsichtlich Größe und Richtung in solchen Farb- oder Verreibewerken ermöglichen.

Die erfindungsgemäße Vorrichtung läßt sich auch zum Prüfen der verschiedenen Materialien der eingesetzten Flächen verwenden, wofür ein in seinen Eigenschaften bekanntes fließfähiges Prüfmedium eingesetzt wird.

Die Erfindung wird nachfolgend anhand mehrerer in der Zeichnung dargestellten Ausführungsbeispiele näher erläutert. In der Zeichnung zeigen:
- **Figur 1:**: eine schematische Schnittdarstellung durch eine Vorrichtung gemäß der Erfindung;
- **Figur 2:**: eine vergrößerte Darstellung des in Figur 1 mit II bezeichneten Bereiches;
- **Figur 3:**: einen auslenkbaren Meßwertübermittler, der einen Teil der erfindungsgemäßen Vorrichtung bildet in Verbindung mit einem an diesem Meßwertübermittler feststellbaren Kraftdiagramm;
- **Figur 4:**: ein anderes Ausführungsbeispiel einer Vorrichtung nach der Erfindung in Verbindung mit einem Mehfachauftrag des zu untersuchenden Mediums;
- **Figur 5:**: eine Vorrichtung nach der Erfindung mit mehreren Zusatzflächen für die Untersuchung der Vorgänge bei einem Mehrfarbendruck oder einer häufigen Rückspaltwiederholung;
- **Figur 6:**: eine Vorrichtung der Erfindung zur Messung der Beeinflussung des Mediums bei Mehrfachspaltung und durch Einbringen von Zusatzstoffen in das fließfähige Medium;
- **Figur 7:**: eine Doppelanordnung einer Vorrichtung gemäß Figur 1; und
- **Figur 8:**: eine Abänderung der Anordnung gemäß Figur 7.

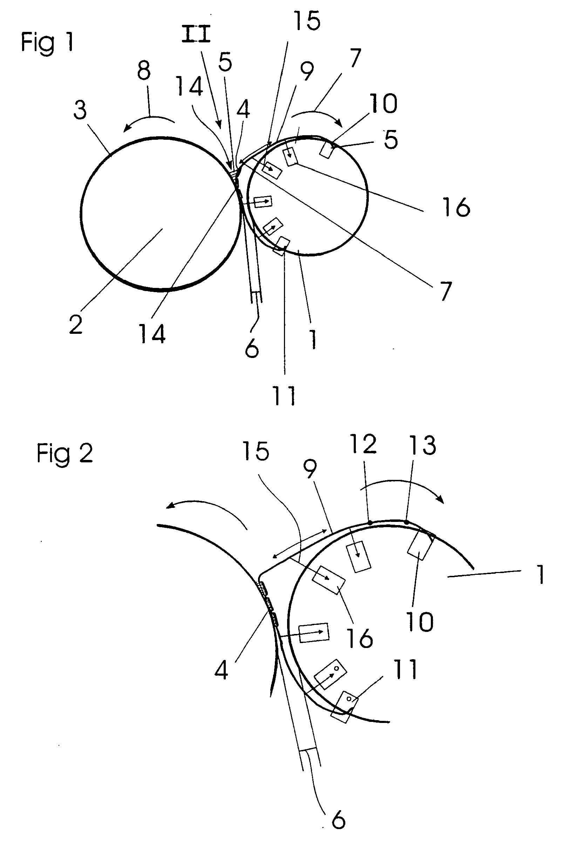

Aus den Figuren 1 und 2 ist das Grundprinzip der Erfindung erkennbar. Diese Vorrichtung weist zwei zylindrische Stützkörper in Form von Walzen 1 und 2 auf, die mit Flächen 3 und 4 versehen sind, zwischen denen das zu untersuchende fließfähige Medium 5 hinsichtlich der Klebrigkeit und sonstiger rheologischer Eigenschaften und insbesondere im Hinblick auf die Zügigkeit dieses Mediums untersucht werden soll. Unter Flächen sind diejenigen Bereiche gemeint, mit denen das fließfähige Medium in Kontakt treten kann, wenn es zwischen den beiden Stützkörpern 1 und 2 in einem einstellbaren Spalt 6 hindurchgequetscht wird. Die beiden Stützkörper 1 und 2 werden im Sinne der Pfeile 7 und 8 gedreht und zwar so, daß die Flächen 3 und 4 mit gleicher Geschwindigkeit bewegt werden, so daß keine Scherkräfte auf das zu untersuchende Medium 5 ausgeübt werden. Die Fläche 3 ist direkt und fest auf dem Stützkörper 2 angeordnet, wobei das diese Fläche 3 bildende Material beispielsweise aus Gummi besteht und einer Auftragswalze in einem Druckwerk nachempfunden ist. Der rotierende Stützkörper 1 trägt einen zunächst eng anliegenden Meßwertübermittler 9, der an seinen Enden in Halterungen 10 und 11 eingespannt ist, die auch als Meßeinrichtungen ausgebildet sein können. Dieser Meßwertübermittler, der ohne fließfähiges Medium 5 eng an dem Stützkörper 1 anliegt, weist zumindest einen dehnbaren Bereich, beispielsweise zwischen den Punkten 12 und 13 auf, um eine Auslenkung bzw. um ein Abheben dieses Meßwertübermittlers 9 vom Stützkörper 1 zu ermöglichen. In diesem Meßwertübermittler 9 sind Bereiche ausgebildet, die Teile einer Fläche 4 bilden, die mit dem zu untersuchenden Medium 5 in Kontakt gebracht werden soll, um die im divergierenden Spaltbereich 14 beim Spalten des fließfähigen Mediums auftretenden Kräfte zu messen. Diese aus den einzelnen Abschnitten gebildete Fläche 4 besteht aus dem Material, welches bei der bestimmungsgemäßen Anwendung des fließfähigen Mediums in Berührung kommt. Dies ist beispielsweise beim Drucken der Fall, wo die Farbe auf Papier aufgebracht wird. Die Flächenteile 4 stellen also beispielsweise Papier dar. In Verbindung mit der beispielsweise aus Gummi bestehenden Fläche 3 sind hier die Verhältnisse beim Bedrukken von Papier praxisgerecht nachgestellt, so daß die bei einem solchen Druckvorgang auftretenden Spaltkräfte tatsächlich gemessen werden können. Beim Drehen des Stützkörper 1 und 2 in Richtung der Pfeile 7 und 8 wird das fließfähige Medium 5 im divergierenden Spaltbereich 14 aufgerissen, wodurch eine Kraft entsteht, die an den Flächen 4 angreifend den Meßwertübermittler 9 vom Stützkörper 1 abhebt. Wie insbesondere aus der etwas größeren Darstellung in Figur 2 ersichtlich, ist der Meßwertübermittler 9 über mechanische Verbindungsteile, beispielsweise Fäden 15, mit Meßeinrichtungen 16 verbunden, die im Inneren des Stützkörpers 1 angeordnet sind und sich mit diesem und somit auch mit der am Meßvorgang beteiligten Fläche 4 mitbewegen. Aus der unterschiedlichen Auslenkung der mechanischen Verbindungen 15 und auch aus der jeweiligen Richtung läßt sich die beim Spaltvorgang oder beim Ablösevorgang auftretende Kraft hinsichtlich ihrer Größe und Richtung bestimmen. Aus diesen Darstellungen in Figur 1 und 2 ist ersichtlich, daß zwei Flächen, die mit dem Medium in Berührung stehen, bewegt werden und zwar mit gleicher Geschwindigkeit, wobei zumindest eine Fläche auf einer Kreisbahn bewegt wird. Eine dieser Flächen ist selbst dehnbar oder auf einem dehnbaren Meßwertübermittler 9 angeordnet, wobei diese auslenkbare Fläche bzw. ihr zugeordneter Meßwertübermittler mit Meßvorrichtungen 16 in Verbindung steht, um die beim Spaltvorgang auftretenden Kräfte festzustellen.

Die nachfolgenden Beispiele zeigen verschiedene Anwendungsbereiche, wobei jeweils der oberste Grundsatz darin zu sehen ist, daß die mit dem Medium in Verbindung zu bringenden Flächen aus solchen Materialien bestehen, wie sie auch in der Praxis bei der Anwendung des zu untersuchenden Mediums zum Einsatz kommen. Dies sind bei Druckereimaschinen Gummiwalzen, entsprechend preparierte Metallplatten, Klischees, zu bedruckende Papiere oder sonstige zu bedruckende Materialien.

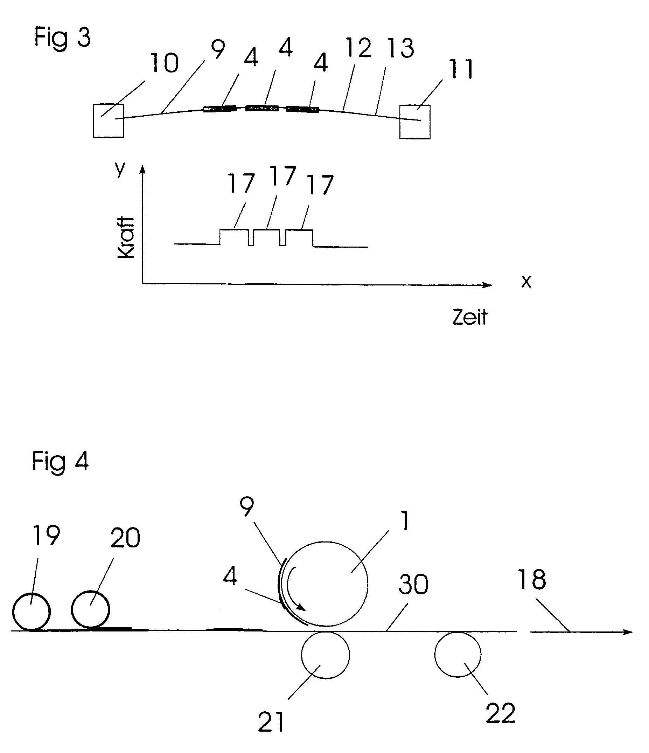

Figur 3 zeigt einen Meßwertübermittler 9, der an den Enden in Halterungen 10 und 11 eingespannt ist sowie mit dem Meßwertübermittler unmittelbar verbundene Flächen 4. Diese Flächen sind entweder aus einem Material, das nicht dehnbar oder ganz allgemein für die Meßwertübertragung nicht geeignet ist. Hierfür ist der Meßwertübermittler 9 vorgesehen, der einen dehnbaren Bereich zwischen den Punkten 12 und 13 aufweist. Selbstverständlich kann der Meßwertübermittler 9 auch insgesamt dehnbar sein. Es ist aber auch möglich, daß die Haltevorrichtung 10 und 11 über eine Feder mit einem undehnbaren Meßwertübermittler verbunden ist. In welcher Form der dehnbare Bereich ausgebildet ist und ob eventuell mehrere dehnbare Bereiche vorgesehen sind, ist zweitrangig, denn wesentlich ist die Tatsache, daß die Fläche bzw. der mit ihr verbundene Meßwertübermittler vom Stützkörper beim Bewegen desselben abgehoben werden kann. Diese Abhebebewegung wird dann, wie bereits erläutert, auf die entsprechenden Meßeinrichtungen übertragen. In Figur 3 ist auch ein Kraftdiagramm über einen Zeitverlauf zu sehen, wobei die jeweiligen Signale 17 beim Auslenken eines Flächenbereiches 4 entstehen. Aus diesen Signalen 17 ist sowohl die Kraftgröße als auch die Dauer derselben erfaßbar und ablesbar.

Figur 4 zeigt eine Vorrichtung, bei der auf einem drehbaren Stützkörper 1 ein Meßwertübermittler 9 mit einer Fläche 4 angeordnet ist. Mit dieser Fläche wirkt diesmal keine rotierende, sondern eine ebene Fläche 30 zusammen, die in Richtung des Pfeiles 18 bewegbar ist. Auf dieser Fläche 30, die die gleiche Geschwindigkeit aufweist wie die Fläche 4, werden durch zwei hintereinander geschaltete Auftragswalzen 19 und 20 jeweils gleiche oder unterschiedlich zu untersuchende Medien aufgetragen, um das Verhalten bei zwei übereinander gelagerten Schichten gleicher oder unterschiedlicher Medien zu untersuchen. Die bewegbare Fläche 30 ist durch eine Stützrolle 21 abgestützt. Eine weitere Stützrolle 22 ist für den Fall vorgesehen, daß ein elastischer Gegendruck zum umlaufenden Stützkörper 1 erwünscht ist und dieser zwischen den beiden Stützrollen 21 und 22 angeordnet wird. Die Erfassung der Meßwerte durch das aus Stützkörper 1, Meßwertübermittler 9 und Fläche 4 gebildete Meßwerk erfolgt in gleicher Weise wie im Zusammenhang mit den Figuren 1 und 2 beschrieben.

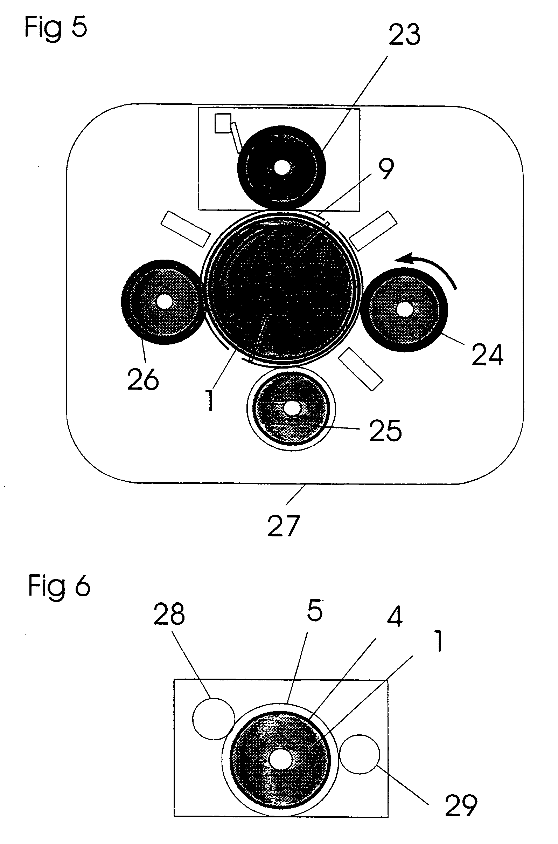

Die in Figur 5 dargestellte Vorrichtung umfaßt ein aus Stützkörper 1, Meßwertübermittler 9 und denen im Inneren des Stützkörpers 1 angeordneten Meßeinrichtungen aufgebautes Meßwerk sowie mehrere am Umfang dieses Meßwerkes gleichmäßig verteilter, weiterer kreiszylindrischer Flächen in Form von angetriebenen Walzen 23 bis 26, denen je nach Anwendungsfall unterschiedliche Aufgaben zukommen. Im ersten Anwendungsfall kann über die Walze 23 das zu untersuchende, fließfähige Medium aufgebracht werden und die übrigen Walzen 24 bis 26 mit ihren kreiszylindrischen Flächen dienen zur Erzeugung aufeinanderfolgender Rückspaltvorgänge, d.h. das durch die Walze 24 und ihre zugeordnete kreiszylindrische Fläche aufgebrachte und gespaltene Medium wird durch die nachfolgenden Walzen 25 und 26 wiederum gespalten. Durch diese mehrfachen Spaltwirkungen wird die rheologische Eigenschaft des zu untersuchenden, fließfähigen Mediums verändert. Mit Hilfe des im Zentrum angeordneten Meßwerkes ist es möglich die unterschiedlichen Auswirkungen dieser mehrfachen Rückspaltwiederholungen festzustellen und auszuwerten. Die gesamte Anordnung ist in einem mit 27 bezeichneten Gehäuse untergebracht, das hinsichtlich Temperatur und Feuchtigkeit klimatisiert werden kann und auch als Schutz dafür dient, daß die zu untersuchenden Medien nicht in die Umgebung ausdampfen können.

Figur 6 zeigt ein zentrales Meßwerk mit einem Stützkörper 1, einer Fläche 4, wie es auch in Figur 1 dargestellt ist, wobei diesem Meßwerk zwei kreiszylindrische Flächen 28 und 29 zugeordnet sind, über die dem Medium 5 ein weiterer Stoff zugeführt wird, um die Änderungen der rheologischen Eigenschaften bei Veränderung des ursprünglich aufgebrachten Mediums 5 durch Hinzugabe weiterer Stoffe zu untersuchen. In der Praxis ist eine solche Anordnung dann gefragt und bedeutungsvoll, wenn der Einfluß der Zugabe von Wasser zu einer Druckfarbe untersucht werden soll, wie dies beispielsweise beim Offsetdruck der Fall ist, wo Wasser als Trennmittel dient und aufgrund seiner Anwesenheit selbstverständlich einen Einfluß auf die Klebrigkeit bzw. einen Einfluß auch auf die Zügigkeit der Druckfarbe ausübt.

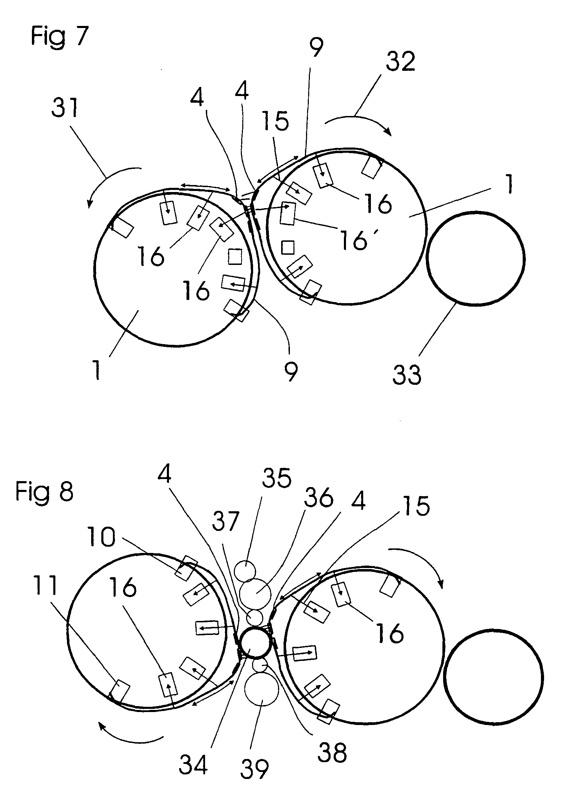

Figur 7 zeigt die Zuordnung von zwei identischen Meßwerken, wobei jedes Meßwerk einen Stützkörper 1, einen Meßwertübermittler 9, darauf integrierte Flächen 4 und im Inneren des Stützkörpers 1 angeordnete Meßeinrichtungen 16 aufweist. Ein zwischen dem Meßwertaufnehmer 9 und ihre zugeordneten Flächen 4 eingebrachtes, fließfähiges Medium führt bei der Drehung der Meßwerke in Richtung der Pfeile 31 und 32 zu einem Abheben der Meßwertübermittler 9 und damit zu einer Anzeige an den jeweiligen Meßeinrichtungen 16. Diese Anordnung ist insbesondere dann vorteilhaft, wenn durch Verwendung von Flächen 4, die keinen Einfluß auf das fließfähige Medium haben, die diesem fließfähigem Medium innewohnende Klebrigkeit und im speziellen auch die ursprünglich innewohnende Zügigkeit festgestellt werden soll. Dabei wird die Zügigkeit aus dem Kraftwert abgeleitet, der senkrecht zur mitwirkenden Fläche auftritt, was beispielsweise bei den mit 16' bezeichneten Meßeinrichtungen der Fall ist. Die übrigen Meßeinrichtungen dienen zur Ermittlung weiterer rheologischer Eigenschaften, beispielsweise der Viskosität des zu untersuchenden fließfähigen Mediums. Eine weitere Walze 33 ist vorgesehen, um beispielsweise die Auswirkungen eines bereits zwischen den Flächen 4 gespaltenen Materials beim Auftragen auf die Walze zu erfassen, die beispielsweise eine Papieroberfläche aufweisen kann. Die Walze kann auch das fließfähige Medium tragen.

Bei der Anordnung nach Figur 8 sind zwei Meßwerke entsprechend Figur 7 vorgesehen, die einen größeren Abstand aufweisen und zwischen sich einen Walzenkörper 34 aufnehmen, der mit den jeweiligen Flächen 4 der beiden Meßwerke über ein eingebrachtes, fließfähiges Medium in Verbindung steht. Dieses wird über mehrere Walzen 35, 36 und 37 auf die Walze 34 aufgetragen und zwar in einem solchen Überfluß, daß es über weitere Walzen 38 und 39 abfließt, oder gerade so viel wie bei der Walze 39 abgenommen wird. Die mit den beiden Meßwerken in Verbindung stehende Walze 34 ist vorgesehen, um in einem solchen Farbfluß bei einem Farbwalzwerk den Einfluß des Hindurchfließens der Farbe in einem solchen Walzwerk auf die jeweilige rheologischen Eigenschaften zu bestimmen. Dabei kann, wie dies beim Verreiben der Farben auch in der Praxis in einem Druckwerk erfolgt, die Walze 34 changierende Bewegungen ausführen, d.h. Bewegungen in Richtung ihrer Längsachse, wodurch Scherkräfte auf das fließfähige Medium zwischen der Walze 34 und den Flächen 4 der Meßwerke erzeugt werden.

Diese Scherkräfte verändern selbstverständlich die rheologischen Eigenschaften des Mediums, wobei die beiden Meßwerke in der Lage sind diese so geänderten Eigenschaften meßtechnisch zu erfassen und an computergestützte Auswerteeinrichtungen weiterzugeben.

## Patentansprüche

1. Verfahren zur Messung der Klebrigkeit eines fließfähigen Mediums, insbesondere einer Druckfarbe, das als Schicht zwischen zwei in gleicher Richtung bewegbaren Belägen durch eine Konvergenzzone, eine Druckzone und eine Divergenzzone geführt wird, wobei als Maß für die Klebrigkeit diejenige Kraft mittels einer Meßvorrichtung meßbar ist, die notwendig ist, um die Beläge voneinander zu trennen, **dadurch gekennzeichnet, daß** mindestens ein Belag (4) auf einer kreiszylindrischen Bahn geführt ist, daß beide Beläge (3, 4) in gleicher Richtung und mit der gleichen Geschwindigkeit angetreiben werden, daß beide Beläge (3, 4) zumindest abschnittsweise diejenigen Materialien enthalten, aus denen die bei der Verarbeitung des zu prüfenden Mediums in bestimmungsgemäßen Vorrichtungen, beispielsweise Druckmaschinen, einander zugeordneten Belägen bestehen, daß mindestens einer (4) der beiden Beläge der Meßwertübertragung dienend direkt oder über einen Meßwertübermittler (9) auf einem Stützkörper (1) angeordnet ist, mindestens einen definiert dehnfähigen Bereich aufweist und mit Meßeinrichtungen (16) für die Feststellung der Größe und der Richtung derjenigen Kraft verbunden ist, mit der einer der Beläge (3) vom Stützkörper (1) unter Dehnung des Dehnbereiches (12, 13) beim Lösen oder Spalten der an mindestens einem der Beläge (4) anhaftenden Schicht abgehoben wird und daß die Meßeinrichtungen (16) mit dem bewegten Belag (4) synchron mitbewegt wird.

2. Verfahren nach Anspruch 1, **dadurch gekennzeichnet, daß** beide Beläge (3, 4) auf kreiszylindrischer Bahn geführt sind.

3. Verfahren nach Anspruch 1 oder 2, **dadurch gekennzeichnet, daß** die Beläge (3, 4) in ihrem gegenseitigen Abstand einstellbar sind.

4. Verfahren nach einem der Ansrpüche 1 bis 3, **dadurch gekennzeichnet, daß** die Bewegungsgeschwindigkeit der Beläge (3, 4) einstellbar ist.

5. Vorrichtung zur Messung der Klebrigkeit eines fließfähigen Mediums, insbesondere einer Druckfarbe, das als Schicht zwischen zwei in gleicher Richtung bewegbaren Belägen durch eine Konvergenzzone, eine Druckzone und eine Divergenzzone führbar ist, wobei als Maß für die Klebrigkeit diejenige Kraft mittels einer Meßvorrichtung meßbar ist, die notwendig ist, um die Beläge voneinander zu trennen, wobei die Vorrichtung aus zwei Stützkörpern (1,2), zwei auf den Stützkörpern angeordneten Belägen (3,4), Messeinrichtungen (16) und den Stüztkörpern zugeordneten Antriebs mitteln besteht, wobei mindestens ein Belag (3,4) (4) auf einem kreiszylindrischen Stützkörper angeordnet ist, beide Beläge (3, 4) in gleicher Richtung und mit der gleichen Geschwindigkeit angetrieben werden, beide Beläge (3, 4) zumindest abschnittsweise diejenigen Materialien enthalten, aus denen die bei der Verarbeitung des zu prüfenden Mediums in bestimmungsgemäßen Vorrichtungen, beispielsweise Druckmaschinen, einander zugeordneten Belägen bestehen, mindestens einer (4) der beiden Beläge der Meßwertübertragung dienend direkt oder über einen Meßwertübermittler (9) auf einem Stützkörper (1) angeordnet ist, mindestens einen definiert dehnfähigen Bereich aufweist und mit Meßeinrichtungen (16) für die Feststellung der Größe und der Richtung derjenigen Kraft verbunden ist, mit der einer der Beläge (3) vom Stützkörper (1) unter Dehnung des Dehnbereiches (12, 13) beim Lösen oder Spalten der an einem der Beläge (4) anhaftenden Schicht abhebbar ist und daß die Meßeinrichtungen (16) in mindestens einem Stützkörper (1,2) angeordnet und mit dem bewegten Belag (4) synchron mitbewegbar sind.

6. Vorrichtung nach Anspruch 5, wobei beide Beläge (3, 4) auf kreiszylindrischer Bahn führbar sind.

7. Vorrichtung nach Anspruch 5 oder 6, wobei die Beläge (3, 4) in ihrem gegenseitigen Abstand einstellbar sind.

8. Vorrichtung nach einem der Ansrpüche 5 bis 7, wobei die Bewegungsgeschwindigkeit der Beläge (3, 4) einstellbar ist.

9. Vorrichtung nach Anspruch 5 bis 8, wobei mehrere bewegbare Beläge (23 bis 26; 28, 29) dem die Kräfte aufnehmenden und auf Meßeinrichtungen übermittelnden Belag (4) zugeordnet sind.

10. Vorrichtung nach Anspruch 9, wobei die weiteren zugeordneten Beläge (23 bis 26; 28, 29) kreiszylindrisch ausgebildet sind.

11. Vorrichtung nach einem der Ansprüche 5 bis 10, wobei die Meßeinrichtungen (16) zur Messung der Kraft zwischen den Belägen (3, 4) hinsichtlich Größe und Richtung mechanisch (15) mit dem Belag (4) oder dem Meßwertübermittler (9) in Verbindung stehen.

12. Vorrichtung nach Anspruch 11, wobei mehrere mechanische Verbindungen (15) mit dem Belag (4) oder dem Meßwertübermittler (9) mit Abstand zueinander in Bewegungsrichtung gesehen mit mehreren Meßeinrichtungen (16) ausgebildet sind.

13. Vorrichtung nach einem der Ansprüche 5 bis 12, wobei eine optische Meßeinrichtung innerhalb eines Stützkörpers (1) zur Festlegung der räumlichen Auslenkung des Meßwertübermittlers (9) vorgesehen ist.

14. Vorrichtung nach einem der Ansprüche 5 bis 13, wobei zwei kreiszylindrische Stützkörper (1) mit auslenkbaren Belägen und einem Meßwertübermittler (9), die zusammen mit den Meßwerteinrichtungen (16) ein Meßwerk bilden, vorgesehen sind, die zwischen sich das zu untersuchende Medium aufnehmen.

15. Vorrichtung nach Anspruch 14, wobei der auslenkbare Belag (4) aus einem das Medium unbeeinflußenden Material besteht.

16. Vorrichtung nach Anspruch 14 oder 15, wobei zwischen den beiden aus Stützkörper (1) und Meßwertübermittler (9) ausgebildeten Meßwerken eine Walze (34) angeordnet ist, die mit beiden Meßwerken zusammenwirkt und zusätzlich in ihrer Achsrichtung hin- und herverschiebbar antreibbar ist.

## Claims

1. A method for measuring the stickiness of a flowable medium, in particular a printing ink, which is led as a layer between a first and a second surface that can be moved in the same direction, through a convergence zone, a pressure zone and a divergence zone, it being possible to use a measuring device to measure, as a measure of the stickiness, that force which is needed to separate the surfaces from each other, **characterized in that** the first surface (4) is led on a circulary cylindrical path, **in that** both surfaces (3,4) can be driven positively in the same direction and at the same speed, **in that** both surfaces (3,4) contain, at least in some sections, materials of which actual material with surfaces of an intended device for processing of the flowable medium are composed, **in that** the first material surface (4) used to transfer the force which is necessary to seperate the surface from each other is arranged on a supporting body, directly or via a measured-value transmitter(9), has at least one area of set extensibility and is connected to measuring device (21) for establishing the magnitude and the direction of that force with which one of the material with surface (3) is lifted off the supporting body (1), extending the extensible area (12,13), during the separation or splitting of the layer adhering at minimum one of material surface (4), and **in that** the measuring devices is moved synchronously together with the material with surface.

2. The methode as claimed in claim 1, **characterized in that** both materials with surfaces (3,4) are led on a circularly cylindrical path.

3. The methode as claimed in claim 1 or 2,
**characterized in that** a distance between the surfaces (3, 4) can be adjusted.

4. The methode as claimed in one of claims 1 to 3,
**characterized in that** a speed of movement of the material with surfaces (3,4) can be adjusted.

5. A device for measuring the stickness of a flowable medium, in particular a printing ink, which is led as a layer between a first and a second surface that can be moved in the same direction, through a convergence zone, a pressure zone and a divergence zone, it being possible to use a measuring device to measure, as a measure of the stickiness, that force which is needed to seperate the surfaces from each other, arranged in a way that the material with surfaces are led on a circulary cylindrical path, in that both material surfaces (3,4) can be driven positively in the same direction and at the same speed, in that both materials (3,4) contain, at least in some sections, materials of which actual surfaces of an intended device, for example printing machines, for prozessing of the flowable medium are composed, in that one (4) material surfaces used to transfer the force which is necessary to separate the surfaces from each other is arranged on a supporting body (1), directly or via a measured-value transmitter (9), has at least one area of set extensibility and is connected to measuring device (16) for establishing the magnitude and the direction of that force with which one of the material surface (3) is lifted off the supporting body (1), extending the extensible area (12,13), during the separation or splitting of the layer adhereing to one of the material surfaces and that the device is arranged of two supporting bodies (1,2), two on the supporting bodies arranged materials (3,4) and measuring devices (16) with assigned driving devices, whereby at least one material is arranged with at least one supporting body (1,2) being moved synchronously with the material (4).

6. The device as claimed in claim 5, whereby
both surfaces (3,4) are led on a circularly cylindrical path.

7. The device as claimed in claim 5 or 6, whereby
the distance between the surfaces (3, 4) can be adjusted.

8. The device as claimed in claim 5 or 7, whereby
the speed of movement of the surfaces (3, 4) can be adjusted.

9. The device as claimed in claim 5-8, whereby
the number of movable surfaces (23 to 26;28,29) are assigned to the first surface (4), which senses the forces and transmits them to the measuring devices.

10. The device as claimed in claim 9, whereby
the number of movable surfaces (23-26;28,29) are circularly cylindrical.

11. The device as claimed in one of claims 5...10, whereby the measuring devices (16) for measuring the magnitude and direction of the force between the material (3,4) are connected mechanically (15) to the material (4) or to the measured-value transmitter (9).

12. The device as claimed in claim 11, whereby
the number of mechanical connections (15) to the first surface (4) or to the measured-value transmitter (9), at a distance from one another as viewed in the direction of movement, are formed by a number of measuring devices (16).

13. The device as claimed in one of claims 5 to 12,
whereby an optical measuring device is provided inside a supporting body (1) to detect the physical deflection of the measured-value transmitter (9).

14. The device as claimed in one of claims 5 to 13
whereby two circulary cylindrical supporting bodies (1) with deflectable material and a measured-value transmitter (9) are provided and, together with the measuring devices (16), from a measured mechanism, the said surfaces holding the flowable medium between them.

15. The device as claimed in claim 14, whereby
the material with surface (4) consist of a material that does not influence the flowable medium.

16. The device as claimed in claim 14 or 15, whereby between the measuring device (1) and the measuring mechanism(9), there is a roll(34) arranged which interacts with both measuring mechanisms and, in addition, can be driven displaceably to and fro in its axial direction.

## Revendications

1. Procédé de mesure de l'adhésivité d'une substance coulante, notamment d'une encre d'imprimerie, qui est guidée sous forme de couche entre deux garnitures déplaçables dans le même sens à travers une zone de convergence, une zone de pression et une zone de divergence, la force qui est nécessaire pour séparer l'une de l'autre les garnitures pouvant être mesurée en tant que mesure de l'adhésivité au moyen d'un dispositif de mesure, **caractérisé en ce qu'**au moins une garniture (4) est guidée sur une piste cylindrique circulaire, **en ce que** les deux garnitures (3, 4) sont entraînées dans le même sens et à la même vitesse, **en ce que** les deux garnitures (3, 4) contiennent au moins en partie les matériaux qui constituent les garnitures associées les unes aux autres lors du traitement de la substance d'essai dans des dispositifs appropriés, par exemple des imprimantes, **en ce qu'**au moins l'une (4) des deux garnitures servant au transfert des valeurs de mesure est disposée directement ou par le biais d'un dispositif de transfert des valeurs de mesure (9) sur un corps de support (1), présente au moins une région extensible de manière définie et est connectée à des dispositifs de mesure (16) pour déterminer l'intensité et le sens de la force avec laquelle l'une des garnitures (3) est soulevée du corps de support (1) par extension de la région extensible (12, 13) lors du desserrage ou de la séparation de la couche adhérant sur au moins l'une des garnitures (4) et **en ce que** les dispositifs de mesure (16) sont entraînés de manière synchrone avec la garniture déplacée (4).

2. Procédé selon la revendication 1, **caractérisé en ce que** les deux garnitures (3, 4) sont guidées sur une piste cylindrique circulaire.

3. Procédé selon la revendication 1 ou 2, **caractérisé en ce que** l'écartement mutuel des garnitures (3, 4) peut être ajusté.

4. Procédé selon l'une quelconque des revendications 1 à 3, **caractérisé en ce que** la vitesse de déplacement des garnitures (3, 4) est ajustable.

5. Dispositif de mesure de l'adhésivité d'une substance coulante, notamment d'une encre d'imprimerie, qui peut être guidée sous forme de couche entre deux garnitures déplaçables dans le même sens à travers une zone de convergence, une zone de pression et une zone de divergence, la force qui est nécessaire pour séparer l'une de l'autre les garnitures pouvant être mesurée en tant que mesure de l'adhésivité au moyen d'un dispositif de mesure, le dispositif se composant de deux corps de support (1, 2), de deux garnitures (3, 4) disposées sur les corps de support, de dispositifs de mesure (16) et de moyens d'entraînement associés aux corps de support, au moins une garniture (3, 4) étant disposée sur un corps de support cylindrique circulaire, les deux garnitures (3, 4) étant entraînées dans le même sens et à la même vitesse, les deux garnitures (3, 4) contenant au moins en partie les matériaux qui constituent les garnitures associées les unes aux autres lors du traitement de la substance d'essai dans des dispositifs appropriés, par exemple des imprimantes, au moins l'une (4) des deux garnitures servant au transfert des valeurs de mesure étant disposée directement ou par le biais d'un dispositif de transfert des valeurs de mesure (9) sur un corps de support (1), présentant au moins une région extensible de manière définie et étant connectée à des dispositifs de mesure (16) pour déterminer la valeur et le sens de la force avec laquelle l'une des garnitures (3) peut être soulevée du corps de support (1) par extension de la région extensible (12, 13) lors du desserrage ou de la séparation de la couche adhérant sur l'une des garnitures (4) et les dispositifs de mesure (16) étant disposés dans au moins un corps de support (1, 2) et pouvant être entraînés de manière synchrone avec la garniture déplacée (4).

6. Dispositif selon la revendication 5, dans lequel les deux garnitures (3, 4) peuvent être guidées sur une piste cylindrique circulaire.

7. Dispositif selon la revendication 5 ou 6, dans lequel l'écartement mutuel des garnitures (3, 4) peut être ajusté.

8. Dispositif selon l'une quelconque des revendications 5 à 7, dans lequel la vitesse de déplacement des garnitures (3, 4) est ajustable.

9. Dispositif selon l'une quelconque des revendications 5 à 8, dans lequel plusieurs garnitures déplaçables (23 à 26 ; 28, 29) sont associées à la garniture (4) recevant les forces et les transmettant aux dispositifs de mesure.

10. Dispositif selon la revendication 9, dans lequel les autres garnitures associées (23 à 26 ; 28, 29) sont réalisées sous forme cylindrique circulaire.

11. Dispositif selon l'une quelconque des revendications 5 à 10, dans lequel les dispositifs de mesure (16) pour la mesure de la force entre les garnitures (3, 4) en terme d'intensité et de sens sont en liaison mécanique (15) avec la garniture (4) ou avec le dispositif de transfert des valeurs de mesure (9).

12. Dispositif selon la revendication 1, dans lequel plusieurs liaisons mécaniques (15) avec la garniture (4) ou le dispositif de transfert des valeurs de mesure (9) sont réalisées à distance les uns des autres vu dans le sens de déplacement avec plusieurs dispositifs de mesure (16).

13. Dispositif selon l'une quelconque des revendications 5 à 12, dans lequel un dispositif de mesure optique est prévu à l'intérieur d'un corps de support (1) pour établir la déviation spatiale du dispositif de transfert des valeurs de mesure (9).

14. Dispositif selon l'une quelconque des revendications 5 à 13, dans lequel deux corps de support cylindriques circulaires (1) sont pourvus de garnitures déplaçables et d'un dispositif de transfert des valeurs de mesure (9), qui forment ensemble avec les dispositifs de mesure (16) un système de mesure, qui reçoivent entre eux la substance à tester.

15. Dispositif selon la revendication 14, dans lequel la garniture déplaçable (4) se compose d'un matériau influençant la substance.

16. Dispositif selon la revendication 14 ou 15, dans lequel, entre les deux systèmes de mesure constitués par le corps de support (1) et le dispositif de transfert des valeurs de mesure (9), est disposé un rouleau (34), qui coopère avec les deux systèmes de mesure et qui peut en outre être entraîné de manière déplaçable d'avant en arrière dans sa direction axiale.
